# EUROPÄISCHE PATENTSCHRIFT

(11) **EP 1 093 773 B1**
(45) Veröffentlichungstag und Bekanntmachung des Hinweises auf die Patenterteilung: **04.10.2001**
(21) Anmeldenummer: 99121052.7
(22) Anmeldetag: 21.10.1999
(51) Int. Cl.: A61F 2/08, A61B 17/58

(54) **Biodegradierbarer Fixationskörper**
Biodegradable fixation device
Corps de fixation biodégradable

(43) Veröffentlichungstag der Anmeldung: 25.04.2001
(73) Patentinhaber: Karl Storz GmbH & Co. KG, 78532 Tuttlingen (DE)
(72) Erfinder: Strobel, Michael,Dr.med., 94315 Straubing (DE); Weiler, Andreas, Dr., 10550 Berlin (DE); Timmermans, André, 7261 Ruurlo (NL)
(74) Vertreter: Weller, Wolfgang, Dr.

(56) Entgegenhaltungen:
- WO-A-93/15694
- WO-A-99/44544
- FR-A- 2 744 010
- US-A- 5 084 050

## Beschreibung

Die Erfindung betrifft einen biodegradierbaren Fixationskörper, insbesondere zum Verankern von Implantaten an Knochen, mit einem Körper, an dessen Außenseite Verankerungselemente angeordnet sind.

Ein biodegradierbarer Fixationskörper in Form einer Interferenzschraube ist aus OP-JOURNAL 14 (1998) S. 278-284 "Biodegradierbare Interferenzschrauben in der Kreuzbandchirurgie" A. Weiler et al., Georg Thieme Verlag Stuttgart, New York bekannt.

Biodegradierbare Materialien sind solche, die vollständig vom Körper aufgenommen werden, es fallen darunter auch Composite-Materialien, die nur zu einem gewissen Anteil vom Körper aufgenommen werden.

Interferenzschrauben dienen dazu, ein Sehnentransplantat oder ein Sehnenimplantat in einem Knochen zu verankern. Dazu wird in den Knochen ein Kanal gebohrt, in den das Sehnentransplantat eingezogen wird. Die Interferenzschraube ist nun dazu vorgesehen, in einen Zwischenraum zwischen Sehnentransplantat und der Innenwand des Bohrkanales eingedreht zu werden, so daß dann das Sehnentransplantat zwischen der Schraube und der Innenwand des Bohrkanales geklemmt ist. Da auf eine solche Sehne, beispielsweise bei einem Kreuzbandersatz in einem Kniegelenk, erhebliche Kräfte einwirken, muß die Klemmkraft entsprechend groß sein, um eine ortsfeste sichere Verankerung sicherzustellen. Dazu ist die Interferenzschraube mit Verankerungselementen in Form eines Außengewindes versehen, das sich in das Knochenmaterial an der Innenseite des Bohrkanales hineinfrißt. Gleichzeitig tritt das Außengewinde auch in Eingriff mit dem zu verankernden Sehnentransplantat.

Um die erheblichen mechanischen Klemmkräfte ausüben zu können, muß der Fixationskörper eine bestimmte geometrische Mindestausdehnung haben.

Wird nun der Fixationskörper nach und nach biologisch abgebaut, entsteht dann dementsprechend ein relativ großer Hohlraum. In diesen Hohlraum kann zwar nach und nach Knochenmaterial einwachsen, so daß das Transplantat nach wie vor fest verankert bleibt. Verläuft der biologische Abbau nicht parallel mit dem Knochenwachstum, können instabile Verhältnisse dahingehend entstehen, daß das nachgewachsene Knochenmaterial noch nicht in ausreichendem Maße oder noch nicht ausreichend verfestigt ist, aber schon Hohlräume aufgrund des biologischen Abbaus entstanden sind.

Ein weiterer Nachteil besteht darin, daß der relativ große innere Hohlraum erst dann durch Knochenmaterial auszufüllen ist, wenn die Wand des Körpers ausreichend biologisch abgebaut ist, damit überhaupt ein Zutritt zu diesem Bereich für das Knochenwachstum ermöglicht ist.

Müssen nun Revisionen vorgenommen werden und soll ein neues Transplantat eingesetzt werden, müssen entsprechend wieder Bohrungen zum Einsetzen des Fixationskörpers vorgesehen werden. Ist aber nicht genügend Knochenmaterial vorhanden, können diese Bohrungen nicht einwandfrei angebracht werden und dementsprechend das neue Transplantat nicht fixiert werden.

Es ist daher Aufgabe der Erfindung, einen biodegradierbaren Fixationskörper der eingangs genannten Art zu schaffen, der den Aufbau eines knöchernen Netzwerkes im Bereich der Transplantatstelle ermöglicht.

Erfindungsgemäß wird die Aufgabe dadurch gelöst, daß der Körper mit zahlreichen Perforationen versehen ist, über die Knochenmaterial in das Innere des Körpers einwachsen kann.

Die Perforationen ermöglichen ein knöchernes Einwachsen von Knochenmaterial auch in das Innere des Fixationskörpers, so daß der gesamte Fixationskorpus knöchern durchwachsen wird. Resorbiert sich nun das Material, so erhält man ein knöchernes Netzwerk, welches ausreichend stabil ist und auch im Falle von Revisionen nach mißlungenem Verankern des Transplantates oder nach einer erneuten Ruptur des Transplantates keine Probleme im Revisionsfall bereitet. Es steht also auch schon dann, wenn das biodegradierbare Material noch nicht vollständig resorbiert ist, schon ein ausreichender Knochenkörper zur Verfügung, um nicht nur eine besonders stabile Verankerung des Transplantates auf Dauer aufrechtzuerhalten, sondern daß schon genügend Knochenmaterial zum Einbringen weiterer Bohrungen vorhanden ist. Verläuft das Knochenwachstum schneller als die Bioresorption des Materials, kann schon Material über die Perforationen in den inneren Hohlraum einwachsen oder mit darin vorhandenem Knochenmaterial verwachsen und ein stabiles, festes, dreidimensionales Netzwerk aufbauen. Bei massiven Körpern können die Perforationen teilweise oder ganz durch den Körper hindurchreichen. Die Form, die Verteilung und die Anzahl der Perforationen kann variiert werden.

WO-A-99 44544 zeigt die Merkmale des Oberbegriffs des Anspruchs 1.

In einer weiteren Ausgestaltung der Erfindung sind die Perforationen als durchgehende Löcher in einer Wand eines Hohlkörpers ausgebildet.

Diese Maßnahme hat den Vorteil, daß die Perforationen herstellungstechnisch einfach zu bewerkstelligen sind, die Stabilität des Körpers aber dennoch aufrechterhalten werden kann.

In einer weiteren Ausgestaltung der Erfindung sind die Perforationen zwischen den Verankerungselementen ausgebildet.

Diese Maßnahme hat den Vorteil, daß die Verankerungselemente sehr stabil bzw. massiv ausgebildet sein können, um die entsprechenden Verankerungskräfte ausüben oder ertragen zu können und nicht durch Perforationen geschwächt sind.

In einer weiteren Ausgestaltung der Erfindung, bei der der Körper als Schraubenkörper einer Interferenzschraube ausgebildet ist und die Verankerungselemente in Form eines Außengewindes ausgebildet sind, sind die Perforationen zwischen den Gängen des Außengewindes ausgebildet. Dadurch werden die zuvor erwähnten Vorteile bei einer solchen Ausgestaltung erzielt.

In einer weiteren Ausgestaltung der Erfindung weist der Körper einen durchgehenden Kanal auf.

Diese Maßnahme hat den Vorteil, daß der Kanal, beispielsweise durch entsprechende Konturierung, einerseits als Aufnahme für ein Werkzeug zum Eindrehen eines als Interferenzschraube ausgebildeten FixatonsKörpers dienen kann. Das Knochenmaterial kann aber andererseits auch über die Mündungen des Kanales in den Innenraum hineinwachsen. In den Kanal können darüber hinaus Knochenmaterialien eingebracht werden, z.B. Knochenschlamm oder Knochenteile, die beispielsweise aus der Beckenkammregion entnommen worden sind, die dann alsbald mit den durch die Perforation einwachsenden Knochenzellen einen festen Knochenverbund ausbilden. Es ist auch möglich, in diesem Kanal Antizytostatika oder Antibiotika zu deponieren, um Entzündungen zu verhindern. Es können auch Wachstumsfaktoren (Hormone etc.) im Hohlraum eingelagert werden. Diese können auch bei der Fertigung auf der Oberfläche des Körpers abgeschieden werden oder in die degrabierbare Masse eingebettet werden.

Es versteht sich, daß die vorstehend genannten und die nachstehend noch zu erläuternden Maßnahmen nicht nur in den angegebenen Kombinationen, sondern auch in anderen Kombinationen oder in Alleinstellung einsetzbar sind, ohne den Rahmen der vorliegenden Erfindung zu verlassen. Die Erfindung wird nachfolgend anhand eines ausgewählten Ausführungsbeispiels in Zusammenhang mit den beiliegenden Zeichnungen näher beschrieben und erläutert.

Es zeigen:
- Fig. 1: eine Seitenansicht eines erfindungsgemäßen biodegradierbaren Fixationskörpers in Form einer Interferenzschraube, wobei die rechte Hälfte als Längsschnitt dargestellt ist,
- Fig. 2: eine teilweise geschnittene Ansicht, wobei dargestellt ist, wie der Fixationskörper gerade zum Verankern eines Transplantates in einen Bohrkanal eines Knochens mit Hilfe eines Werkzeuges eingetrieben wird, und
- Fig. 3: eine der Fig. 2 vergleichbare Darstellung nach vollständigem Eindrehen des Fixationskörpers mit verankertem Transplantat.

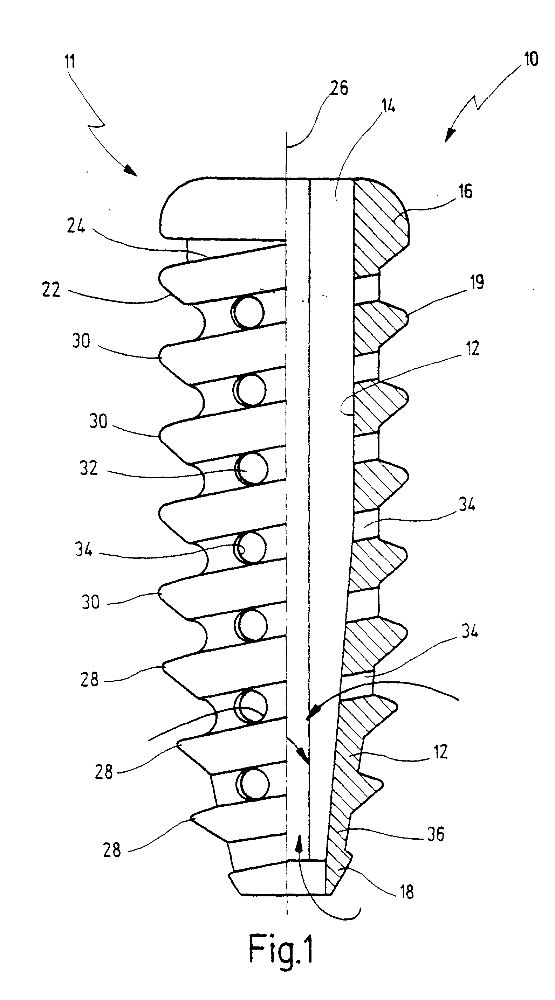

Ein in der Fig. 1 dargestellter Fixationskörper ist in seiner Gesamtheit mit der Bezugsziffer 10 versehen.

Der Fixationskörper 10 ist im dargestellten Ausführungsbeispiel eine Interferenzschraube 11.

Die Interferenzschraube 11 weist einen Körper 12 auf, der ein Kopfende 16 und ein Eintreibende 18 aufweist. Der etwa hohlzylindrische Schraubenkörper verjüngt sich im Bereich des Eintreibendes 18.

Durch den Körper 12 reicht vom Kopfende 16 bis zum Eintreibende 18 ein durchgehender Kanal 14, dessen Querschnitt, zumindest im Bereich des Kopfendes 16, sechseckförmig konturiert ist.

An der Außenseite ist der Körper 12 mit Verankerungselementen 19 in Form eines Außengewindes 20 versehen, das vom Eintreibende 18 bis zum Kopfende 16 reicht.

Das Außengewinde 20 ist im dargestellten Ausführungsbeispiel als Sägengewinde profiliert. Die jeweils vorlaufende bzw. dem Eintreibende 18 zugewandte Flanke 22 steht zur Mittellängsachse 26 der Interferenzschraube 11 unter einem Winkel von etwa 45 Grad. Die andere Flanke 24 läuft, vom Kopfende 16 zum Eintreibende 18 gesehen, um etwa 15 Grad nach unten geneigt, gegenüber einer senkrecht zur Achse 26 verlaufenden Ebene. Im Bereich des Eintreibendes 18 bis in etwa zum maximalen Kerndurchmesser des Körpers 12 gehen die Flanken 22 bzw. 24 über eine scharfe Spitze 28 ineinander über.

In den nachlaufenden Abschnitten gehen die Flanken 22 und 24 jeweils über eine stumpfe Spitze 30 ineinander über.

Daraus resultiert ein erster Bereich des Außengewindes 20 in der Nähe des Eintreibendes 18 mit scharfem Gewinde, an den sich ein Bereich, der sich bis zum Kopfende 16 erstreckt, mit stumpfem Gewinde anschließt.

Die Interferenzschraube 11 besteht aus einem biodegradierbarem Material. Beispiele biodegradierbarer Materialien sind: Polycaprolactone, Poly(L-Laktide), Polyglykolide, Poly(D,L-Laktide), Poly(D,L-Laktid-co-Glykolide), Poly(D,L-Laktid-co-Caprolactone) Polydioxanone, Copolyoxalate, Polycarbonate, z.B. Polyglykolidco-Trimethylencarbonat, Poly(Glutamin-co-Leucine).

Die Wand 36 des Körpers 12 ist mit zahlreichen Perforationen 32 in Form von durchgehenden Löchern 34 versehen.

Die Löcher 34 sind dabei so angeordnet, daß sie zwischen den Gängen des Außengewindes 20 angeordnet sind.

Im Bereich des Eintreibendes 18 sind bis zum ersten vollständig umgelaufenen Gewindegang noch keine Perforationen vorgesehen, da dort der Körper 12 ohnehin verjüngt aufgebaut ist und somit eine unnötige Schwächung durch die Perforationen erfolgen könnte. Im weiteren Verlauf sind jeweils etwa sechs Löcher 34 pro Gewindeumlauf vorgesehen.

Die Löcher 34 im dargestellten Ausführungsbeispiel sind kreisrund und regelmäßig voneinander beabstandet angeordnet.

Die Form, die Verteilung und die Anzahl der Perforationen ist jeweils so gewählt, daß eine ausreichende Stabilität des Körpers erhalten bleibt, dennoch zahlreiche Durchwachsstellen vorhanden sind.

Wie das in Fig. 1 durch Pfeile dargestellt ist, kann Knochenmaterial durch die zahlreichen Perforationen 32 bzw. Löcher 34 in das Innere des Körpers 12 hineinwachsen. Im Bereich des Kopfendes 16 bzw. des Eintreibendes 18 ist dies auch durch die entsprechenden Mündungsöffnungen des mittigen Kanals 14 möglich.

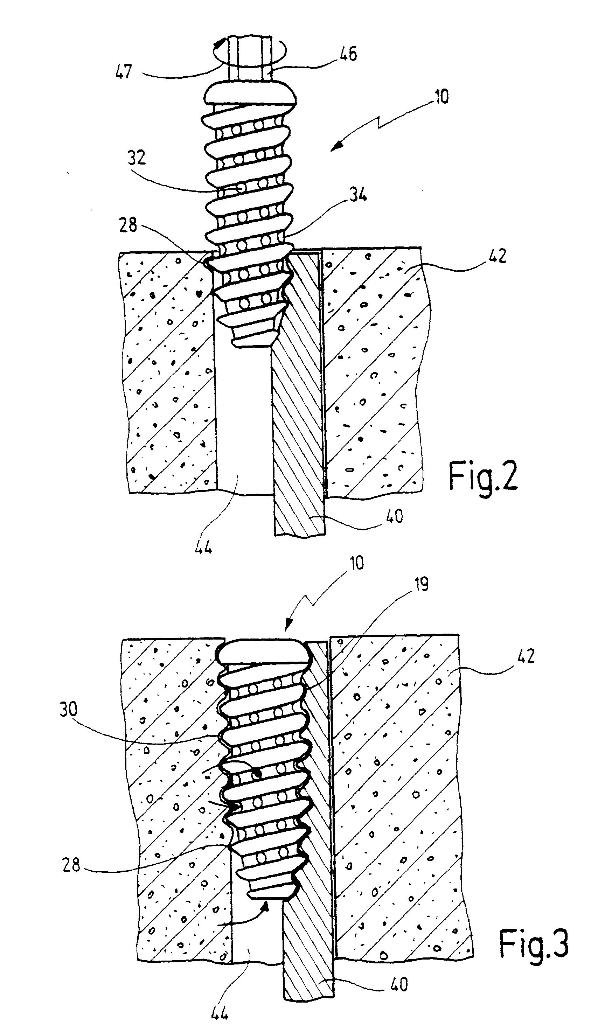

Der Vorgang einer Interferenzverschraubung läuft, wie in den Fig. 2 und 3 dargestellt, folgendermaßen ab. In einem Knochen 42, in dem ein Sehnentransplantat 40 verankert werden soll, wird eine Öffnung in Form eines durchgehenden Bohrkanales 44 bewerkstelligt. Der Durchmesser des Bohrkanales 44 wird so gewählt, daß in diesen das Sehnentransplantat 40 bzw. ein Ende davon eingeschoben werden kann.

Beim Ersatz eines Kreuzbandes werden entsprechende Bohrkanäle 44 sowohl im Femur als auch in der Tibia bewerkstelligt und dort jeweils das Sehnentransplantat verankert, wie das beispielsweise in Abb. 1 des vorgenannten Artikels im OP-JOURNAL 14 (1998) a.a.O. gezeigt ist.

Nach Einziehen des Sehnentransplantates 40 in den Bohrkanal 44 wird die Interferenzschraube 11 so angesetzt, daß sich deren Eintreibende 18 zwischen Innenwand des Bohrkanales 44 und das äußere Ende des Sehnentransplantates 40 schiebt. Dieses Einsetzen und Einschieben wird durch eine Verjüngung im Bereich des Eintreibendes 48 begünstigt.

In das Kopfende 16 ist ein Werkzeug 46 eingeschoben, dessen Außenkontur der Querschnittskontur des inneren Kanales 14 entspricht, beispielsweise einer sechseckförmigen Querschnittskontur. Durch Drehen des Werkzeuges 46, wie das durch einen Pfeil 47 angedeutet ist, kann die Interferenzschraube 11 eingedreht werden.

In Fig. 2 ist eine Situation dargestellt, bei der die scharfen Spitzen 28 des Außengewindes 20 im Bereich des Eintreibendes 18 gerade mit der Innenwand des Bohrkanales 44, also mit dem Knochenmaterial, in Eingriff treten. Diese scharfen Spitzen 28 schneiden beim Eindrehen der Interferenzschraube 11 eine relativ schmale scharfkonturierte schraubenförmige Linie an der Innenseite des Bohrkanales 44 ein. Beim weiteren Eindrehen der Interferenzschraube 11 laufen dann die nachfolgend stumpfen Gewindeabschnitte in das von den scharfen Spitzen 28 vorgeschnittene Innengewinde ein, erweitern diese und sorgen für die eigentliche radiale Kompression. Dabei graben sich die stumpfen Gewindeabschnitte, wie das in Fig. 3 auf der rechten Seite ersichtlich ist, entsprechend tief und fest in das Material des Sehnentransplantates 40 hinein.

Durch die Perforationen 32 bzw. die Löcher 34 kann nun Knochenmaterial in den Innenraum des Körpers 12 nach und nach hineinwachsen.

Der Innenraum kann mit Knochenmaterial, z.B. Knochenschlamm oder Knochenteilen, die beispielsweise aus der Beckenkammregion des Patienten entnommen wurden, aufgefüllt sein. Dadurch bildet sich alsbald, z.B. nach sechs bis acht Wochen, ein fester Verbund aus Knochenmaterial mit dem Innenraum der Interferenzschraube. Das biodegradierbare Material wird nach und nach abgebaut, so daß dann in die dadurch entstandenen Hohlräume weiteres Knochenmaterial einwachsen kann.

Durch diese vorteilhafte Ausgestaltung muß der zeitliche Ablauf der Biodegradation nicht mit der Knochenwachstumsgeschwindigkeit korreliert sein, dieses kann insbesondere viel rascher ablaufen, da dem Knochenmaterial über die Perforationen 32 ausreichend Platz zur Verfügung steht, schon vor oder während des biologischen Abbaus eine dreidimensional verästelte Knochenstruktur auszubilden.

## Patentansprüche

1. Biodegradierbarer Fixationskörper, insbesondere zum Verankern von Transplantaten (40) an Knochen (42), mit einem Körper (12), an dessen Außenseite Verankerungselemente (19) angeordnet sind, wobei der Körper (12) mit zahlreichen Perforationen (32) versehen ist, über die Knochenmaterial in das Innere des Körpers (12) einwachsen kann, **dadurch gekennzeichnet, daß** der Körper (12) als steifer Hohlkörper ausgebildet ist, **daß** ferner die Perforationen (32) als durchgehende Löcher (34) in einer Wand (36) des Hohlkörpers (12) ausgebildet sind, und **daß** die Perforationen (32) zwischen den Verankerungselementen (19) ausgebildet sind.

2. Biodegradierbarer Fixationskörper nach Anspruch 1, **dadurch gekennzeichnet, daß** er als Schraubenkörper einer Interferenzschraube (11) ausgebildet ist, bei dem die Verankerungselemente (19) in Form eines Außengewindes (20) ausgebildet sind, wobei zwischen den Gängen des Außengewindes (20) die Perforationen (32) ausgebildet sind.

3. Biodegradierbarer Fixationskörper nach Anspruch 1 oder 2, **dadurch gekennzeichnet, daß** der Körper (12) einen durchgehenden Kanal (14) aufweist.

## Claims

1. Biodegradable fixation element, in particular for anchoring transplants (40) on bones (42), comprising a body (12) having anchoring members (19) arranged on its outer side, said body (12) is provided with numerous perforations (32) through which bone material can grow into the interior of the body, **characterized in that** the body (12) is designed as a rigid hollow body, said perforations (32) are formed as holes (34) passing completely through a wall (36) of the hollow body (12), and **in that** these perforations (32) are formed between the anchoring members (19).

2. Biodegradable fixation element according to claim 1, **characterized in that** it is formed as a screw body of an interference screw (11) upon which said anchoring members (19) are provided in the form of an outer threading (20), said perforations (32) are formed between windings of the outer threading (20).

3. Biodegradable fixation element according to claim 1 or 2, **characterized in that** the body (12) comprises a channel (14) passing completely therethrough.

## Revendications

1. Corps de fixation biodégradable, notamment pour l'ancrage d'implants (40) à des os (42), comportant un corps (12) sur le côté extérieur duquel sont disposés des éléments d'ancrage (19), le corps (12) étant pourvu de nombreuses perforations (32) par lesquelles la matière de l'os peut croître à l'intérieur du corps (12), **caractérisé en ce que** le corps (12) est conçu en tant que corps creux rigide, **en ce qu'**en outre les perforations (32) sont pratiquées en tant que trous traversants (34) dans une paroi (36) du corps creux (12), et **en ce que** les perforations (32) sont pratiquées entre les éléments d'ancrage (19).

2. Corps de fixation biodégradable selon la revendication 1, **caractérisé en ce qu'**il est conçu en tant que corps de vis d'une vis d'interférence (11), dans lequel les éléments d'ancrage (19) sont réalisés sous la forme d'un filetage extérieur (20), les perforations (32) étant pratiquées entre les filets du filetage extérieur (20).

3. Corps de fixation biodégradable selon la revendication 1 ou 2, **caractérisé en ce que** le corps (2) comporte un canal (14) le traversant de part en part.
